# EUROPEAN PATENT APPLICATION

(11) **EP 3 943 067 A1**
(43) Date of publication of application: **26.01.2022**
(21) Application number: 21187458.1
(22) Date of filing: 23.07.2021
(51) Int. Cl.: A61K 9/00, A61K 31/00, A61K 36/06, A61K 36/07, A61K 36/258, A61K 47/00, A61P 39/00

(54) **A FORMULATION FOR USE IN THE TREATMENTOF RADIOTHERAPY-DERIVED SIDE EFFECTS**

(30) Priority: 23.07.2020 IT 202000017914
(71) Applicant: Meti Biosolutions S.r.l., 15067 Novi Ligure (IT); BTGin Co., Ltd., Daejeon 34024 (KR)
(72) Inventor: GRAMAGLIA, Pietro Paolo, 20161 MILANO (IT)
(74) Representative: Lunati & Mazzoni S.r.L.

(57) **Abstract**

It is provided a formulation for use in the treatment of radiotherapy derived side effects comprising a mixture of triterpene saponins, beta-glucan, and chitooligosaccharides, wherein the mixture of triterpene saponins is extracted from ginseng, is complexed in a matrix including the beta-glucan and the chitooligosaccharides and comprises substituents in the C3 position of the type chosen from monosaccharide, disaccharide or hydroxyl, substituents in the C6 position comprising monosaccharides and / or disaccharides, and substituents in the C20 position comprising monosaccharides or hydroxyls.

## Description

The present invention relates to a formulation for use in the treatment of radiotherapy derived side effects of the type specified in the preamble of the first claim.

In particular, the present invention relates to a formulation for use in the treatment of radiotherapy derived side effects, such as RIM (Radiation - induced mucositis), or rather mucositis induced by radiation; the formulation can be in the state of viscous liquid or solid, for example made in tablets or capsules, and can be able to carry out, simultaneously or alternatively, its beneficial effects both at a topical and systemic level.

The term RIM has been defined as a side effect of radiotherapy in cancer patients, and is described as the onset of a lesion in healthy tissue, lasting between 7 and 98 days, resulting in acute inflammation of the mucous membranes, for example of the tongue and pharynx, following exposure to radiation therapy.

In this document, the term RIM is used as a generic term to indicate a plurality of pathologies or inflammatory effects caused by radiotherapy, which in case of involvement of the upper aerodigestive tract are defined as "oral mucositis" and indicated with the acronym RIOM (radiation induced oral mucositis), in the case of lesions in the small intestine they are called "gastrointestinal mucositis" and in the case of lesions affecting the rectal mucosa they are called "proctitis".

The progression of RIM can lead to a life-threatening acute stage, for example due to physical obstruction of food and water intake in the case of RIOM, resulting in cachexia and septic complications due to loss of physical barrier provided by the protective epithelium and basement membrane.

A plethora of medical devices and drugs are currently known to be used to combat RIM at various levels.

These medical devices and drugs are used from the prevention of its onset to the modulation of the symptoms related to it highly impacting on the quality of life of patients, and can be topically applied agents (local anaesthetics, capsaicin, sodium alginate, saliva spray, honey), agents adopted systemically (sucralfate) and agents that reduce the microbial load (chlorhexidine, chamomile, povidone-iodine).

The known art described includes some important drawbacks.

In particular, some treatments designed for the prevention of RIM are characterized by acting on a limited number of factors that characterize the pathogenesis of RIM. Others act either exclusively on pain or on the reduction/removal of ROS (reactive oxygen species), or mechanically as a barrier for the mucous membranes (sprays of saliva, honey, sucralfate), and finally as antibacterial, antiseptic and antifungal agents (chlorhexidine, chamomile, povidone-iodine).

Again, the limit of the use of antibiotics is given by the risk of the onset of resistance to them, and it is an intervention that is limited to treating one of the effects caused by radiotherapy, that is, immunosuppression.

In this situation, the technical task underlying the present invention is to devise a formulation for use in the treatment of side effects deriving from radiotherapy capable of substantially obviating at least part of the aforementioned drawbacks. One of the purposes of the present invention is to provide a product that acts on several levels on the RIM, given the complex pathobiology.

Still, an object of the present invention is to use substances for the final composition of the formulation characterized by a poly-functional profile as regards the targets involved in RIM.

One purpose of the formulation according to the present invention is to provide a final composition of the formulation whose substances provide, as a whole, a preventive action against damage from radiotherapy.

A further object of the present invention is to provide a formulation whose substances provide, as a whole, a radiation protection effect.

A further object is to provide a formulation whose substances as a whole provide a mechanical barrier function for the mucous membranes.

In addition, an object is to provide a formulation whose substances as a whole provide a bactericidal effect.

Furthermore, an object of the present invention is to provide a formulation whose substances provide, as a whole, a topical action as modulators of inflammation.

A further object of the present invention is to provide a formulation whose substances provide, as a whole, a topical action for the reduction of oxidative damage.

Furthermore, it is an object of the present invention to provide a formulation whose substances provide, as a whole, a topical and/or systemic action of protection from damage to the DNA of cells of healthy tissues.

Finally, it is an object of the present invention to provide a formulation whose substances provide, as a whole, a systemic action as radio-sensitizing, immunostimulants, anti-angiogenic and anti-inflammatory.

The technical task and the specified aims are achieved by a formulation for use in the treatment of radiotherapy derived side effects as claimed in the attached claim 1.

Preferred technical solutions are highlighted in the dependent claims.

In the present document, the measurements, values, shapes and geometric references (such as perpendicularity and parallelism), when associated with words like "about" or other similar terms such as "approximately" or "substantially", are to be considered as except for measurement errors or inaccuracies due to production and/or manufacturing errors, and, above all, except for a slight divergence from the value, measurements, shape, or geometric reference with which it is associated. For instance, these terms, if associated with a value, preferably indicate a divergence of not more than 10% of the value.

Moreover, when used, terms such as "first", "second", "higher", "lower", "main" and "secondary" do not necessarily identify an order, a priority of relationship or a relative position, but can simply be used to clearly distinguish between their different components.

Unless otherwise specified, as results in the following discussions, terms such as "treatment", "computing", "determination", "calculation", or similar, refer to the action and/or processes of a computer or similar electronic calculation device that manipulates and/or transforms data represented as physical, such as electronic quantities of registers of a computer system and/or memories in, other data similarly represented as physical quantities within computer systems, registers or other storage, transmission or information displaying devices.

The measurements and data reported in this text are to be considered, unless otherwise indicated, as performed in the International Standard Atmosphere ICAO (ISO 2533:1975).

In this text, references to triterpene saponins, dammarane, ginsenosides or mixtures thereof refer to enzymatic and non-enzymatic extracts of ginseng or to extracts deriving from fermentation processes of ginseng or, even more in detail, to enzymatic and non-enzymatic extracts. Korean red ginseng enzymatic or referring to extracts deriving from fermentation processes of Korean red ginseng and reaction by-products of such mixtures such as, for example, by-products of a dehydration or deglycosylation reaction of molecules forming part of said mixtures.

The formulation for use in the treatment of side effects resulting from radiotherapy preferably comprises triterpene saponins, beta-glucan and chito-oligosaccharides. Preferably, the saponins are made from ginseng, in particular preferably Korean red ginseng.

Preferably, the saponins are mixtures comprising substituents in the C3 position of the type chosen from monosaccharide, disaccharide or hydroxyl, substituents in the C6 position comprising monosaccharides and/or disaccharides, substituents in the C20 position comprising monosaccharides or hydroxyls.

Among the possible combinations, for example, the saponins can include substituents in the C3 position of the disaccharide or hydroxyl type, substituents in the C6 position comprising monosaccharides and/or disaccharides and substituents in the C20 position comprising hydroxyls; or they can include substituents in the C3 position comprising monosaccharides or hydroxyls, substituents in the C6 position comprising monosaccharides and/or disaccharides and substituents in the C20 position comprising monosaccharides or hydroxyls.

The aforementioned mixtures can be made by means of an enzymatic process, such as for example according to the method patented by BTGin and described in the patent KR-B-101336646.

Of course, other methods capable of producing enzymatic and non-enzymatic extracts of ginseng and/or Korean red ginseng could be used equivalently, or fermentation methods that respond to the aforementioned characteristics.

The formulation for use in the treatment of side effects resulting from radiotherapy may include, in particular, dammarane, beta-glucan and chito-oligosaccharides. Dammarane can be made from ginseng or, even more specifically, from Korean red ginseng.

Preferably, the dammarane are mixtures comprising substituents in the C3 position of the disaccharide or hydroxyl type, substituents in the C6 position comprising monosaccharides and/or disaccharides and substituents in the C20 position comprising hydroxyls; or mixtures obtained from Korean red ginseng, with substituents in the C3 position comprising monosaccharides or hydroxyls, substituents in the C6 position comprising monosaccharides and/or disaccharides and substituents in the C20 position comprising monosaccharides or hydroxyls. The aforementioned mixtures can also be made by means of an enzymatic process, such as for example according to the method patented by BTGin and described in patent KR-B-101336646.

Of course, other methods capable of producing enzymatic and non-enzymatic extracts of ginseng and/or Korean red ginseng could be used equivalently, or fermentation methods that respond to the aforementioned characteristics.

Even more in detail, the formulation for use in the treatment of side effects resulting from radiotherapy may include ginsenosides, beta-glucan and chito oligosaccharides.

Ginsenosides can be derived from ginseng or, even more specifically, from Korean red ginseng.

Preferably, the ginsenosides are mixtures with substituents R1 comprising disaccharides or hydroxyls, substituents R2 comprising monosaccharides and/or disaccharides and substituents R3 comprising hydroxyls; or mixtures obtained from Korean red ginseng, with R1 substituents comprising monosaccharides or hydroxyls, R2 substituents comprising monosaccharides and/or disaccharides and R3 substituents comprising monosaccharides or hydroxyls.

The aforementioned mixtures can also be made by means of an enzymatic process, such as for example according to the method patented by BTGin and described in patent KR-B-101336646.

Of course, other methods capable of producing enzymatic and non-enzymatic extracts of ginseng and/or Korean red ginseng could be used equivalently, or fermentation methods that respond to the aforementioned characteristics. Preferably, moreover, the mixture of triterpene saponins, preferably in particular dammarane, still more preferably ginsenosides, is complexed in a matrix including at least the beta-glucan.

The matrix also further comprises chito-oligosaccharides.

Preferably, according to the present invention, the formulation includes chito-oligosaccharides in particular obtained from a deacetylation.

Deacetylation is, in particular, suitable for determining the formation of chito-oligosaccharides with a degree of deacetylation lower than 95%.

Even more in detail, the deacetylation is suitable to determine chito-oligosaccharides with a degree of deacetylation between 80% and 90%.

Even more conveniently, the degree of deacetylation of the chito-oligosaccharides is about 90%.

In one embodiment, the formulation can be in the form of a viscous liquid. Alternatively, the formulation can be made in solid form.

The latter embodiment is for example preferable to be administered orally according to the type of RIM to be addressed.

Furthermore, the formulation can allow the realization of a medical device, characterized by a liquid-viscous form, suitable for rectal administration for the treatment of proctitis induced by radiotherapy.

Still, for example, a food supplement or food for special medical purposes can be produced in oral solid form of the formulation according to the present invention suitable for oral administration for the treatment of gastrointestinal mucositis induced by radiotherapy.

The beta-glucan used in the formulation according to the present invention can preferably be obtained by extraction or other methods, from fungi, such as for example from Aureobasidium pullulans, or from yeasts, or from still others.

The formulation according to the invention allows to make particular foods for medical purposes.

In fact, a food for special medical purposes including the formulation for use in the treatment of side effects deriving from radiotherapy falls within the scope of protection of the present invention.

Furthermore, a food supplement including the formulation for use in the treatment of side effects deriving from radiotherapy falls within the scope of protection of the present invention.

In addition to what has been described above, the mixture of triterpene saponins, or dammarane, or ginsenosides can be subjected to liposomal or nanosomial encapsulation or other types of systems to increase its absorption.

Furthermore, the formulation is preferably carried out by balancing the components as defined below.

Preferably, the mixture of triterpene saponins is less than 50% of the total weight of the formulation.

Even more in detail, the mixture of triterpene saponins is in a percentage between 0.5% and 20% of the total weight of the formulation.

Even more conveniently, the mixture of triterpene saponins is in a percentage equal to approximately 12% of the total weight of the formulation.

Preferably, the beta-glucan is in a percentage between 4% and 70% of the total weight of the formulation.

Even more in detail, beta-glucan is in a percentage between 5% and 50% of the total weight of the formulation.

Even more conveniently, beta-glucan is about 12% of the total weight of the formulation.

Preferably, the chito-oligosaccharides are in a percentage comprised between 0.002% and 10% of the total weight of the formulation.

Even more in detail, the chito-oligosaccharides are in a percentage between 0.004% and 5.5% of the total weight of the formulation.

Even more conveniently, the chito-oligosaccharides are in a percentage equal to approximately 1% of the total weight of the formulation.

Specifically, an example of a formulation could include a mixture characterized by ginsenosides deriving from Korean red ginseng composed of Rg3 (R + S), Rk1, Rg5, Rg2, Rh1 fractions.

Or, the mixture could be composed of Compound-K, Rh2, Rg2 and Rh1 fractions.

Or again, the mixture could be composed of Compound-K, Rh1, PPD, PPT fractions. The formulation according to the invention can therefore be constituted by triterpene saponins, beta-glucan, and chito-oligosaccharides, for example according to the percentages just described. Or the formulation could also include triterpene saponins, beta-glucan, and chito-oligosaccharides and other additives or excipients. Furthermore, the formulation may also be part of a product which includes the formulation, with triterpene saponins, beta-glucan, and chito-oligosaccharides in percentages previously indicated, and other components or additives.

The formulation therefore makes it possible to create, for example, a food for special medical purposes which can consist of the following components: Korean red ginseng enzymatic extract, beta-glucan, chito-oligosaccharides with the addition of glutamine, tocopheryl acetate, lecithin and honey.

Furthermore, the formulation makes it possible to create, for example, a medical device that can be made up of the following components: enzymatic extract of Korean red ginseng, beta-glucan, chito-oligosaccharides with the addition of hyaluronic acid, cera alba, tocopheryl acetate and lecithin.

In addition, the formulation allows to realize, for example, a food supplement which can be made up of the present components: Korean red ginseng enzymatic extract, beta-glucan, chito-oligosaccharides with the addition of tocopheryl acetate and lecithin.

The formulation for use in the treatment of radiotherapy derived side effects according to the invention achieves important advantages.

With reference to the combined effect of the mixture of triterpene saponins or dammarane or ginsenosides, obtained by enzymatic process, with beta-glucan, in the initial phase the mixture of ginsenosides of the Korean red ginseng enzyme extract acts synergistically with beta-glucan inhibiting the production of ROS. Furthermore, beta-glucan reduces chromosomal aberrations and increases the rate of DNA repair in lymphocytes, while the mixture of ginsenosides increases the cellular vitality of healthy cells and protects mitochondria by stabilizing their membrane potential.

The mixture of triterpene saponins or dammarane or ginsenosides is, in addition, able to reduce the activity of the metalloprotease of the matrix-2 (MMP-2) and of the metalloproteases of the matrix-9 (MMP-9).

Furthermore, in the phase of primary damage and amplification of the effects of RIM, the production of pro-inflammatory cytokines is more rapidly and more significantly reduced, as well as the translocation of NK-kB (nuclear factor kappa-light- chain-enhancer of activated B cells) is reduced by the chito-oligosaccharides.

Apoptosis is also reduced by the synergy of the mixture of ginsenosides and chito-oligosaccharides.

Further, in the ulcerative phase of RIM, the same is modulated by the containment in the production of inflammatory cytokines, and the macrophage activation and the development of a trained immunity by beta-glucans together with the action of the chito-oligosaccharides makes the system immune ready to react against secondary infections, where the growth of C. albicans and S. aureus can be effectively contained by chito-oligosaccharides, avoiding antibiotic resistance reactions that can result in an even greater risk for the patient's health.

The healing phase is significantly accelerated by the macrophage stimulus of beta-glucans which, containing in the matrix formed by them a mixture of ginsenosides obtained from Korean red ginseng, stimulate the dermal fibroblasts to synthesize growth factors, thus catalysing a more rapid tissue regeneration.

The formulation thus characterized therefore has a radiation protection and radio-sensitization action, the latter extremely useful for making the radiotherapy protocol effective without having to resort to altered fractionation, and above all without creating side effects and/or toxicity.

Still, a specific advantage of the embodiment in viscous liquid, which can remain on the mucous membranes, is that it has a topical therapeutic action, as a protection of the mucous membranes and a catalyst for the healing of radio-induced lesions, said topical action very useful in cases of RIOM to protect the oral mucous membranes.

Furthermore, since the components of the matrix are metabolically active, the active ingredients that compose it act both through haematological reconstitution and through functional monocyte reprogramming activity, which can monitor immunity while the production of T lymphocytes is compromised by radiotherapy. Advantageously, with reference to the production methods and sources of production, given that the formulations that have seen the direct use of Korean red ginseng on humans provide for the administration of several grams of product per day, the solubility of these quantities of raw material for the realization of a liquid/viscous product, as required for the treatment of RIM, it would fail. The enzymatic treatment of Korean red ginseng allows the adoption of much smaller doses of the active ingredient, thus allowing the same active ingredient to remain in solution in a liquid composition without the formation of precipitates as a product is produced well below the solubility limit of the solute in the given solution. Advantageously, the use of beta-glucan obtained for example through the transmutator Aureobasidium pullulans allows to obtain a high solubility in water and at the same time to boast a branched structure, the latter correlated to its greater metabolic efficacy.

Still advantageously, thanks to the presence of the active ingredients in a matrix with a high permanence on the mucous membranes, the choice of using chito-oligosaccharides with a degree of deacetylation of 90% guarantees their solubility in water, making synergism between the different active ingredients possible.

As regards the treatment of RIOM radiation-induced oral mucositis, the use of a formulation that is present in liquid form is preferable, due to the difficulty of patients suffering from this pathology in being able to assimilate oral solid forms.

The obtainment of a viscous matrix, in which mixtures of triterpene saponins, or dammarane, or ginsenosides are complexed, obtained by enzymatic and non-enzymatic extraction, preferably from Korean red ginseng, is due to the use of beta-glucan, which according to the concentration with which it is used is able to modulate the viscosity of the liquid in which it is dissolved leading to the formation of a solution with a different degree of density.

Furthermore, preferably in the formulation according to the present invention, being able to use a beta-glucan matrix confers a triple advantage. In fact, the matrix allows to obtain an adhesion capacity of the product to the oral and oesophageal mucosa by increasing a topical radiation protection action which otherwise would occur only systemically after ingestion of the product; moreover, where mucosal lesions occur, beta-glucans are able to activate macrophages to produce signal molecules and synthesis of growth factors, leading to cell proliferation, vascularization and contraction of the lesion; in this way the matrix also acts as a carrier for the other active ingredients contained therein, thus providing a long-release effect. Chito-oligosaccharides in addition to providing metabolic effects on immunity, give the compound bactericidal activity and may have a carrier function for the ginsenoside mixture.

Advantageously, this complex of active ingredients acts on several levels on the problems induced by radiotherapy.

In this context, all the details can be replaced by equivalent elements and the materials, shapes and dimensions can be any.

## Claims

1. A formulation for use in the treatment of radiotherapy-derived side effects, comprising a mixture of triterpenoid saponins, beta-glucan, and chitooligosaccharides, **characterised in that**
- said mixture of triterpenoid saponins is extracted from ginseng, is complexed in a matrix including said beta-glucan and said chitooligosaccharides, and comprises:
- substituents at C3 of the type selected from monosaccharide, disaccharide or hydroxyl,
- substituents at C6 comprising monosaccharides and/or disaccharides,
- substituents at C20 comprising monosaccharides or hydroxyls.

2. The formulation according to claim 1, wherein said mixture of triterpenoid saponins is a mixture of dammaranes.

3. The formulation according to any one of the preceding claims, wherein said mixture of triterpenoid saponins is a mixture of ginsenosides.

4. The formulation according to any one of the preceding claims, wherein said mixture of triterpenoid saponins is extracted from Korean red ginseng.

5. The formulation according to any one of the preceding claims, wherein said mixture of triterpenoid saponins is subject to liposomal or nanosomal encapsulation.

6. The formulation according to any one of the preceding claims, wherein said mixture of triterpenoid saponins is in a percentage less than 50% of the total weight of the formulation, said beta-glucan is in a percentage ranging from 4% to 70% of the total weight of the formulation, and said chitooligosaccharides are in a percentage ranging from 0.002% to 10% of the total weight of the formulation.

7. The formulation according to any one of the preceding claims, wherein said chitooligosaccharides are obtained by deacetylation with a degree of deacetylation of less than 95%.

8. The formulation according to any one of the preceding claims, wherein said formulation is in the form of a viscous liquid or a solid.

9. The formulation according to any one of the preceding claims, wherein said beta-glucan is obtained from Aureobasidium pullulans or from yeast.

10. The formulation according to any one of the preceding claims, wherein said mixture of triterpenoid saponins is extracted through an enzymatic process.

11. The formulation according to any one of the preceding claims, wherein said mixture of triterpenoid saponins is extracted through a fermentative process.

12. The formulation according to any one of the preceding claims, wherein said mixture of triterpenoid saponins comprises a by-product of a dehydration or deglycosylation reaction.

13. Food for special medical purposes comprising the formulation for use in the treatment of radiotherapy-derived side effects according to any one of the preceding claims.

14. A dietary supplement comprising the formulation for use in the treatment of radiotherapy-derived side effects according to any one of the preceding claims.

15. A medical device comprising the formulation for use in the treatment of radiotherapy-derived side effects according to any one of the preceding claims.
